# DEMANDE DE BREVET EUROPEEN

(11) **EP 1 430 877 A1**
(43) Date de publication de la demande: **23.06.2004**
(21) Numéro de dépôt: 03293259.2
(22) Date de dépôt: 19.12.2003
(51) Int. Cl.: A61K 7/13

(54) **Composition pour coloration de fibres kératiniques contenant une base d'oxydation et un polymère particulier, procédé de préparation de cette composition et procédé de coloration avec ladite composition**

(30) Priorité: 19.12.2002 FR 0216206
(71) Demandeur: L'OREAL, 75008 Paris (FR)
(72) Inventeur: Samain, Henri, 91570 Bievres (FR)
(74) Mandataire: Dossmann, Gérard

(57) **Abrégé**

L'objet de l'invention concerne une composition pour la teinture d'oxydation des fibres kératiniques, en particulier des fibres kératiniques humaines telles que les cheveux humains contenant au moins une base d'oxydation particulière et au moins un polymère sous forme solubilisée ou dispersée de PM en poids supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :
- d'au moins un agent dispersant constitué par un électrolyte soluble
- et d'au moins un agent empêchant l'augmentation de la viscosité,
ainsi qu'un procédé de coloration d'oxydation utilisant ces compositions.

## Description

L'invention est relative à l'utilisation pour la teinture des fibres kératiniques, en particulier des fibres kératiniques humaines telles que les cheveux humains d'une composition de teinture d'oxydation contenant au moins une base d'oxydation et au moins un polymère particulier, un procédé de préparation de ces compositions, ainsi qu'un procédé de coloration d'oxydation utilisant ces compositions.

Aujourd'hui les produits de coloration capillaire permettent de changer la couleur des cheveux pour quelques heures, quelques jours, quelques semaines, quelques mois avec un retour à l'état initial ou une persistance du résultat, tout en respectant l'intégrité de la fibre.

Plusieurs familles d'agents de coloration ont été développées pour atteindre ces différents objectifs en termes de couleurs et de durabilité. La coloration capillaire permanente est réalisée classiquement en utilisant des précurseurs de colorants d'oxydation généralement appelés "bases d'oxydation". Les précurseurs de colorants d'oxydation sont des composés initialement peu ou pas colorés qui développent leur pouvoir tinctorial au sein du cheveu en présence d'agents oxydants tel que le peroxyde d'hydrogène en conduisant à la formation de composés colorés. La formation de ces composés colorés résulte, soit d'une condensation oxydative des "bases d'oxydation" sur elles-mêmes, soit sur des composés modificateurs de coloration, ou "coupleurs" qui sont généralement présents dans les compositions tinctoriales utilisées en teinture d'oxydation. La variété des molécules mises en jeu, qui sont constituées d'une part par les "bases d'oxydation" et d'autre part par les "coupleurs" permet l'obtention d'une palette très riche en coloris. Selon leur tenacité ou leur rapidité à s'estomper, les colorants d'oxydation utilisés dans les formules de coloration, permettent d'obtenir des montées de couleur puissantes et peu sélectives.

Plusieurs procédés d'obtention de polymères hydrosolubles sous forme de dispersion existent dans l'art antérieur. Le brevet FR 2 815 635 porte sur un procédé de préparation d'une dispersion aqueuse d'un polymère permettant de limiter les variations de viscosité du mélange réactionnel tout au long de la polymérisation.

Le brevet FR 2 812 295 porte sur un procédé de préparation d'un polymère cationique de haut poids moléculaire à base de sel de dialkyle et de diallyle ammonium sous forme de perles par la technique de polymérisation en suspension inverse.

La demande WO 02/40622 porte sur l'utilisation d'une solution aqueuse d'un copolymère cationique à base d'un monomère non-ionique et d'un monomère cationique dans une composition d'un détergent.

La demande FR 2 816 833 décrit l'utilisation en cosmétique d'un polymère hydrosoluble sous forme d'une dispersion susceptible d'être obtenu par polymérisation d'au moins un monomère hydrosoluble comportant au moins une double liaison dans une solution aqueuse saline.

La demanderesse a trouvé de manière surprenante et avantageuse que l'introduction de polymères hydrosolubles particuliers qui sont sous forme dispersée ou sous forme solubilisée dans les compositions de coloration de fibres kératiniques contenant des bases d'oxydation particulières permettait de produire des effets supérieurs aux résultats obtenus avec des compositions similaires ne contenant pas de dispersions de polymères hydrosolubles.

En particulier, il a été observé que l'ajout de ces polymères permet d'obtenir dans ces compositions des baisses de sélectivité, des montées de couleur ainsi que des ténacités supérieures à celles obtenues en l'absence de ce polymère.

Ces compositions de coloration de fibres kératiniques sont de plus capables de produire d'autres effets potentiellement intéressants tels que l'épaississement de la composition à la dilution. Elles permettent ainsi d'obtenir un meilleur effet conditionneur de la fibre.

Par ailleurs la demanderesse a découvert que sous forme dispersée dans la composition tinctoriale, le polymère particulier pouvait protéger les colorants d'oxydation des dégradations liées à des agents extérieurs. Cette protection est encore plus importante si les colorants d'oxydation sont introduits lors de la synthèse des polymères sous forme dispersée. Il convient par ailleurs de noter que si la dilution de la dispersion initiale dans la composition finale est suffisante, elle conduira à la solubilisation des particules polymériques hydrodispersées.

L'invention porte sur une composition pour la teinture des fibres kératiniques, en particulier des fibres kératiniques humaines telles que les cheveux humains contenant au moins une base d'oxydation et au moins un polymère hydrosoluble sous forme dispersée ou sous forme solubilisée.

L'invention concerne également un procédé de préparation des compositions colorantes, des procédés de coloration des fibres kératiniques incorporant ces composés, ainsi que des dispositifs de teinture ou "kits" à plusieurs compartiments.

D'autres objets, caractéristiques, aspects et avantages de l'invention apparaîtront encore plus clairement à la lecture de la description et des divers exemples qui suivent.

La présente invention a pour objet une composition pour la teinture des fibres kératiniques, en particulier des fibres kératiniques humaines telles que les cheveux humains contenant au moins une base d'oxydation et au moins un polymère hydrosoluble sous forme dispersée ou sous forme solubilisée de PM moyen en poids supérieur à 10⁶, ledit polymère étant obtenu et introduit dans la composition sous forme de dispersion issue de la polymérisation dans une solution aqueuse saline d'au moins un monomère hydrosoluble comportant au moins une double liaison en présence :
- d'au moins un agent dispersant constitué par un (poly)électrolyte soluble, et
- d'au moins un agent empêchant l'augmentation de la viscosité,
ladite base d'oxydation étant choisie parmi les paraphénylènediamines substituées, les paraaminophénols substitués, les bases hétérocyliques et leurs sels d'addition avec un acide.

Les paraphénylènediamines substituées de l'invention sont de préférence de formule I 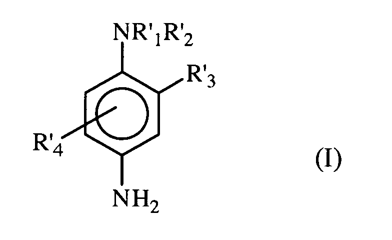 dans laquelle
R'₁ et R'₂ représentent indépendamment l'un de l'autre, un atome d'hydrogène, un radical alkyle linéaire ou ramifié en C₁-C₄, un radical mono ou polyhydroxyalkyle linéaire ou ramifié en C₁-C₄, un radical alcoxy(C₁-C₄)alkyle(C₁-C₄) linéaire ou ramifié, ou forme avec l'atome d'azote les portant un hétérocycle éventuellement substitué par un ou plusieurs groupes hydroxyalkyle en C₁-C₄,

R'₃ et R'₄ représentent indépendamment l'un de l'autre, un atome d'hydrogène, un atome d'halogène, un radical alkyle linéaire ou ramifié en C₁-C₄, un radical mono ou polyhydroxyalkyle linéaire ou ramifié en C₁-C₄, un radical alcoxy en C₁-C₄,
l'un au moins des substituants R'₁ à R'₄ étant différent de l'hydrogène.

Parmi les paraphénylènediamines, on peut citer à titre d'exemple, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Les paraaminophénols substitués de l'invention sont de préférence de formule II 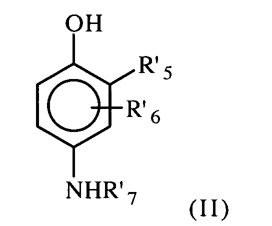 dans laquelle :
R'₅ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
R'₆ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄, ou alcoxy(C₁-C₄)alkyle(C₁-C₄),
R'₇ représente un atome d'hydrogène ou un radical alkyle en C₁-C₄, étant entendu que l'un au moins des radicaux R'₅, R'₆ ou R'₇ de préférence R'₅ ou R'₆, est différent d'un atome d'hydrogène.

Lorsque R'₅ ou R'₆ désigne un radical alkyle en C₁-C₄, il s'agit de préférence d'un radical choisi parmi les radicaux méthyle, éthyle, n-propyle, isopropyle, n-butyle, s-butyle, tert-butyle.

Par "alcoxy (C₁-C₄) ", on entend un radical alkyle(C₁-C₄)-O, le radical alkyle ayant la définition donnée ci-dessus.

Lorsque R'₅ ou R'₆ désigne un halogène, il s'agit de Cl, Br, I ou F.

Plus particulièrement, les composés de formule (II) sont choisis parmi le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition.

De préférence, le N-méthyl-paraaminophénol est utilisé.

Les bases hétérocycliques de l'invention sont notamment choisies parmi les dérivés pyridiniques, les dérivés pyrimidiniques, les dérivés pyrazoliques, et leur sels d'addition avec un acide.

Selon l'invention, la ou les bases d'oxydation de l'invention représentent de préférence, de 0,0005% à 12% en poids environ du poids total de la composition et encore plus préférentiellement de 0,005% à 6% en poids environ. De préférence, les compositions de l'invention contiennent au moins une base hétérocyclique. De préférence, les compositions de l'invention contiennent au moins un coupleur.

Les coupleurs sont choisis parmi ceux classiquement utilisés en coloration d'oxydation, et parmi lesquels on peut citer en particulier les méta-phénylènediamines, les méta-aminophénols, les dérivés mono- ou poly-hydroxylés du naphtalène, le sésamol et ses dérivés , et des coupleurs hétérocycliques notamment choisis parmi les coupleurs indoliques, les coupleurs indoliniques, les coupleurs pyridiniques et leurs sels d'addition avec un acide.

Selon l'invention, le ou les coupleurs représentent de préférence, de 0,0001% à 10% en poids environ du poids total de la composition et encore plus préférentiellement de 0,005% à 5% en poids environ.

D'une manière générale, les sels d'addition avec un acide des bases d'oxydation et coupleurs sont choisis parmi les chlorhydrates, les bromhydrates, les sulfates et les tartrates, les lactates et les acétates.

La partie de la demande EP n° 0 943 628 de l'art antérieur concernant l'obtention du polymère hydrosoluble sous forme d'une dispersion fait partie intégrante de l'invention.

Par polymère hydrosoluble sous forme dispersée ou sous forme solubilisée, on entend au sens de la présente invention un polymère potentiellement hydrosoluble, c'est-à-dire dont la solubilité potentielle dans l'eau est supérieure à 0,1% en poids à 25°C (évaluation visuelle) et qui peut être utilisé in fine sous une forme soluble ou dispersée.

Lesdits polymères utilisés dans le cadre de la présente invention sont avantageusement obtenus à partir de monomères hydrosolubles comportant au moins une double liaison. Ces derniers peuvent être choisis parmi des monomères cationiques, anioniques, non-ioniques et leurs mélanges.

A titre de monomères anioniques, on peut notamment citer l'acide (méth)acrylique, l'acide acrylamido-2-méthylpropanesulfonique, l'acide itaconique et leurs sels d'un métal alcalin, d'alcalinoterreux ou d'ammonium ou ceux issus d'une amine organique telle que l'alcanolamine.

A titre de monomères non-ioniques, on peut notamment citer parmi le (méth)acrylamide, le N-vinylformamide, le N-vinylacétoamide et le (méth)acrylate d'hydroxypropyle.

Les monomères cationiques sont, de préférence, choisis parmi les sels d'ammonium quaternaire dérivés d'une diallylamine, et ceux répondant à la formule suivante (III) : 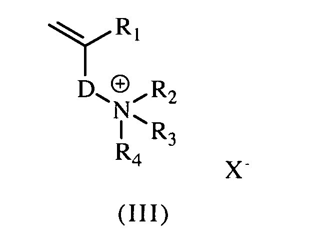 dans laquelle
R₁ représente un atome d'hydrogène ou un groupement méthyle,
R₂ et R₃, identiques ou différents, représentent un atome d'hydrogène ou un groupe alkyle en C₁-C₄ linéaire ou ramifié,
R₄ représente un atome d'hydrogène, un groupe alkyle en C₁-C₄ linéaire ou ramifié ou un groupe aryle,
D représente le motif suivant, 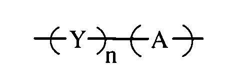 dans lequel :
Y représente une fonction amide, un ester : O-CO ou CO-O, un uréthane ou une urée,
A représente un groupement alkylène en C₁-C₁₀ linéaire ou ramifié, cyclique ou acyclique, pouvant être substitué par un groupement aromatique ou hétéroaromatique. Les groupements alkylènes peuvent être interrompus par un groupement aromatique ou hétéroaromatique divalent, un atome d'oxygène, un atome d'azote, un atome de soufre ou un atome de phosphore ; l'alkylène pouvant contenir une cétone, un amide, un ester : O-CO ou CO-O, un uréthane, ou une urée,
n est un nombre entier variant de 0 à 1,
X⁻ représente un contre ion anionique, comme par exemple un chlorure ou un sulfate.

A titre d'exemples de monomères cationiques hydrosolubles, on peut notamment citer le chlorhydrate ou le sulfate dérivé du (méth)acrylate de diméthylaminoéthyle, le chlorure de (méth)acryloyloxyéthyltriméthylammonium, le chlorure de (méth)acryloyloxyéthyldiméthylbenzylammonium, le chlorhydrate ou le sulfate dérivé du N-[diméthylaminopropyl](méth)acrylamide, le chlorure de (méth)acrylamidopropyltriméthylammonium, le chlorure de (méth)acrylamidopropyldiméthylbenzylammonium, le chlorhydrate ou le sulfate dérivé du (méth)acrylate de diméthylaminohydroxypropryle, le chlorure de (méth)acryloyloxyhydroxypropyltriméthylammonium, le chlorure de (méth)acryloyloxyhydroxypropyldiméthylbenzylammonium et le chlorure de diméthyldiallylammonium.

De préférence, le polymère hydrosoluble selon l'invention est polymérisé à partir d'au moins un monomère cationique.

De préférence, les polymères hydrosolubles sont polymérisés à partir des monomères comportant au moins une double liaison suivants :
- 0 à 30% en moles d'acide acrylique,
- 0 à 95,5% en moles d'acrylamide et,
- 0,5 à 100% en moles d'au moins un monomère cationique représenté dans la formule (III) telle que définie ci-dessus.

Les polymères hydrosolubles sont polymérisés à partir d'un monomère cationique et d'acide acrylique, le nombre de mole du monomère cationique étant supérieur au nombre de mole d'acide acrylique.

Comme polymères hydrosolubles particulièrement préférés dans l'invention, on peut notamment citer ceux polymérisés à partir de
- 10% de chlorure d'acryloyloxyéthyldiméthylbenzylammonium et de 90% d'acrylamide,
- 30% de chlorure d'acryloyloxytriméthylammonium, 50% de chlorure d'acryloyloxyéthyldiméthylbenzylammonium, et de 20 % d'acrylamide,
- 10% de chlorure d'acryloyloxyéthyltriméthylammonium et de 90% d'acrylamide
- 30% de chlorure de diallyldiméthylammonium et de 70% d'acrylamide
- 30% d'acide acrylique et de 70% d'acrylamide.

Les polymères décrits ci-dessus ont un poids moléculaire moyen en poids supérieur à 1 000 000 et de préférence entre 1 000 000 et 50 000 000. Le poids moléculaire est déterminé par la méthode RSV (Reduced Specific Viscocity) telle que définie dans "Principles of Polymer Chemistry" Cornell University Press, Ithaca, NY 1953 Chapter VII "Determination of molecular Weight" pp 266-316.

Dans ces compositions, les polymères de l'invention peuvent être présents à une concentration comprise entre 0,05% et 10%, de préférence entre 0,1% et 5%, et encore plus préférentiellement entre 0,2% et 2% en poids par rapport au poids total de la composition.

L'agent dispersant utilisé dans la présente invention, est un polyélectrolyte, de préférence un polyélectrolyte cationique obtenu par polymérisation de 50 à 100% en moles d'au moins un monomère cationique choisi parmi les sels, par exemple, les chlorhydrates ou les sulfates, dérivés du (méth)acrylate de diméthylaminoéthyle, du N-[diméthylaminopropyl](méth)acrylamide, ou d'une di(méth)allylamine, le chlorure de (méth)acryloyloxyéthyltriméthylammonium, le chlorure de (méth)acrylamidopropyltriméthylammonium, le chlorure de diméthyldiallylammonium, et leurs mélanges, et de 50 à 0% en moles d'acrylamide. On peut utiliser en outre une polyamine telle qu'une polyalkylèneamine.

Selon l'invention, la solution aqueuse saline qui sert de milieu de dispersion au polymère hydrosoluble est une solution aqueuse saline permettant de solubiliser les monomères hydrosolubles comportant des doubles liaisons et l'agent dispersant, mais ne solubilisant pas le polymère hydrosoluble obtenu.

Cette solution aqueuse saline comprend au moins un sel, de préférence un sel anionique divalent tel que, par exemple, le sulfate d'ammonium, l'hydrogénosulfate d'ammonium, le sulfate de sodium, l'hydrogénosulfate de sodium, le sulfate de magnésium, l'hydrogénosulfate de magnésium, le sulfate d'aluminium, l'hydrogénosulfate d'aluminium. Le sulfate d'ammonium et le sulfate de sodium sont particulièrement préférés.

Ces sels peuvent être utilisés en une concentration allant de 15 % en poids jusqu'à leurs concentrations de saturation de manière à ce qu'ils présentent les propriétés mentionnées ci-dessus. La solution aqueuse peut contenir en outre des sels anioniques monovalents, comme le chlorure de sodium et le chlorure d'ammonium.

L'agent empêchant l'augmentation de la viscosité du milieu réactionnel lors de la polymérisation est choisi parmi :
(A). les acides polycarboxyliques ou leurs sels,
(B). les polyphénols,
(C). les composés cycliques contenant un groupe hydroxy et un groupe carboxy, ou leurs sels,
(D). l'acide gluconique ou ses sels,
(E). les produits réactionnels obtenus par réaction d'une méthoxyhydroquinone et/ou d'un monomère cationique (méth)acrylique avec un composé qui génère des radicaux, sous une atmosphère oxydante,
(F). les produits réactionnels obtenus par réaction d'un polymère cationique (méth)acrylique avec un composé qui génère des radicaux, sous une atmosphère oxydante,
(G) les produits réactionnels obtenus par réaction d'un polymère cationique (méth)acrylique avec un oxydant, et
leurs mélanges.

L'utilisation d'un tel milieu réactionnel permet d'obtenir, lorsque le milieu est agité lors de la polymérisation, une dispersion stable de polymère intrinsèquement hydrosoluble.

L'addition d'au moins un agent empêchant l'augmentation de la viscosité (A) à (G), tels que décrits ci-dessus permet d'effectuer la polymérisation des monomères hydrosolubles tels que décrits ci-dessus, avec un agitateur à faible vitesse tout en évitant la formation de particules grossières. Il est préférable que les agents empêchant l'augmentation de la viscosité (A) à (G) soient solubles dans l'eau, mieux encore dissous dans la solution aqueuse saline qui sert de milieu de dispersion.

Comme exemples de composé (A), on peut citer l'acide oxalique, l'acide adipique, l'acide tartrique, l'acide malique, l'acide phtalique et leurs sels.

Comme exemples de composé (B), on peut notamment citer le résorcinol et le pyrogallol.

Comme exemples de composé (C), on peut notamment citer l'acide m-hydroxybenzoïque, l'acide p-hydroxybenzoïque, l'acide salicylique, l'acide gallique, l'acide tannique et leurs sels.

Comme exemples de composé (D), on peut notamment citer le gluconate de sodium, le gluconate de potassium, le gluconate d'ammonium, et différents sels aminés de l'acide gluconique.

Comme exemples de composé (E), on peut notamment citer ceux obtenus en laissant réagir un composé qui génère des radicaux, sous un courant de gaz oxygéné, dans une solution contenant la méthoxyhydroquinone et/ou un monomère cationique (méth)acrylique. Le composé qui génère des radicaux peut être un amorceur de polymérisation couramment utilisé dans la polymérisation radicalaire. On peut notamment citer un amorceur de polymérisation azoïque hydrosoluble tel que le chlorhydrate de 2,2'-azobis(2-amidinopropane), vendu par exemple sous la dénomination V-50 par la société Wako Chemical Industries, ou le chlorhydrate de 2,2'-azobis[2-(2-imidazoline-2-yl)propane] vendu par exemple sous la dénomination commerciale VA-044 par la société Wako Chemical Industries, ou un amorceur de polymérisation de type oxydoréducteur hydrosoluble tel que l'association persulfate d'ammonium/hydrogénosulfite de sodium.

On peut obtenir un agent empêchant l'augmentation de la viscosité (F) par réaction d'un amorceur de polymérisation qui est un composé qui génère des radicaux, sous une atmosphère oxydante, par exemple sous un courant de gaz oxygéné, avec un agent dispersant selon l'invention. L'amorceur de polymérisation peut être un amorceur de polymérisation azoïque hydrosoluble tel que cité ci-dessus, ou un amorceur de polymérisation de type oxydoréducteur hydrosoluble tel que l'association persulfate d'ammonium/hydrogénosulfite de sodium.

L'agent empêchant l'augmentation de la viscosité (G) peut être obtenu sous la forme d'un polymère oxydé de faible masse moléculaire, par oxydation d'un agent dispersant selon l'invention obtenu par polymérisation d'un monomère cationique (méth)acrylique, avec du peroxyde d'hydrogène ou un halogène comme oxydant.

Comme monomères cationiques (méth)acryliques utilisés pour la préparation des agents empêchant l'augmentation de la viscosité (E), (F) et (G), on peut citer, par exemple, le chlorhydrate ou le sulfate dérivé du (méth)acrylate de diméthylaminoéthyle, le chlorure de (méth)acryloyloxyéthyltriméthylammonium, le chlorure de (méth)acryloyloxyéthyldiméthylbenzylammonium, le chlorhydrate ou le sulfate dérivé du N-[diméthylaminopropyl](méth)acrylamide, le chlorure de (méth)acrylamidopropyltriméthylammonium, le chlorure ou le sulfate dérivé du (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium.

Ces agents empêchant l'augmentation de la viscosité (A) à (G) peuvent être utilisés seuls ou en mélange, en une quantité comprise de préférence entre 10 ppm et 10 000 ppm par rapport au poids de la solution réactionnelle.

La composition selon l'invention peut également contenir, en plus des colorants d'oxydation définis ci-dessus, un ou des colorants directs pour enrichir les nuances en reflets. On peut citer notamment tous les colorants aromatiques et/ou non aromatiques d'utilisation courante tels que les colorants nitrés et aminés, les phénols les aminophénols, les naphtols, les méthines, les azométhines, les nitros, les diamines aromatiques, les colorants anthraquinoniques et naphtoquinoniques, les porphyrines, les tétraphénylporphyrines, métalloporphyrines, les phtalocyanines, les cyanines méthiniques, les colorants naturels de type caroténoïdes, flavonoïdes, les molécules fluorescentes (fluorescéine, rhodamine, coumarine...).

Selon l'invention, le ou les colorants directs utilisés selon l'invention représentent de préférence, de 0,001% à 10% en poids environ du poids total de la composition tinctotriale et encore plus préférentiellement de 0,005% à 5% en poids environ.

Le milieu approprié pour la composition pour la coloration de fibres kératiniques est de préférence un milieu aqueux constitué par de l'eau ou par un mélange d'eau et d'un solvant cosmétiquement acceptable, choisi de préférence parmi les alcools inférieurs en C₁-C₄, l'alcool benzylique, les éthers de polyols, les alcanes en C₅-C₁₀, les cétones en C₃-C₄, les acétates d'alkyles en C₁-C₄, le diméthoxyéthane, et leurs mélanges.

Le pH des compositions de l'invention est compris généralement entre 3 et 11, de préférence entre 4 et 10.

Les additifs cosmétiques sont choisis parmi des agents tensio-actifs anioniques, cationiques, non-ioniques ou amphotères, des agents épaississants non polymériques comme des acides ou des électrolytes, des agents antioxydants, des agents dispersants, des agents séquestrants, des parfums, des agents de conditionnement, des agents conservateurs, des agents opacifiants, des agents alcalins, des agents acides ainsi que tout autre adjuvant utilisé habituellement en teinture de matières kératiniques.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20% en poids par rapport au poids total de la composition.

Dans la composition prête à l'emploi ou dans la composition oxydante, l'agent oxydant est choisi parmi le peroxyde d'hydrogène, le peroxyde d'urée, le ferricyanure de métaux alcalins, les persels tels que les borates et les persulfates. L'utilisation du peroxyde d'hydrogène est particulièrement préférée.

Cette composition appliquée sur les cheveux peut se présenter sous des formes galéniques diverses, telles qu'une lotion, une crème, un gel ou sous tout autre forme appropriée pour réaliser une teinture des fibres kératiniques. Elle peut également être conditionnée sous pression en flacon aérosol en présence d'un agent propulseur et former une mousse.

Dans le cas des aérosols, le propulseur utilisé peut être constitué par les gaz liquéfiés ou comprimés usuellement employés pour la préparation de compositions aérosols. On emploiera, de manière préférentielle, l'air, le gaz carbonique, l'azote comprimé ou encore un gaz liquéfiable à température ordinaire tel que le diméthyléther, les hydrocarbures halogénés (fluorés en particulier) ou non, et leurs mélanges.

La présente invention concerne également un procédé de préparation des compositions de l'invention consistant à introduire dans un milieu contenant au moins une base d'oxydation choisie parmi les paraphénylènediamines substituées, les paraaminophénols substitués et les bases hétérocycliques, et au moins une dispersion d'un polymère hydrosoluble de PM moyen en poids supérieur à 10⁶, ladite dispersion étant obtenue par polymérisation dans une solution aqueuse saline d'au moins un monomère hydrosoluble comportant au moins une double liaison en présence :
- d'au moins un agent dispersant constitué par un (poly)électrolyte soluble, et
- d'au moins un agent empêchant l'augmentation de la viscosité.

Une variante de ce procédé consiste à introduire au moins une base d'oxydation selon l'invention au moment de la préparation de la dispersion de polymère et d'introduire la dispersion contenant la ou les bases d'oxydation dans le milieu final de la composition tinctoriale.

La présente invention concerne également un procédé de teinture des fibres kératiniques, et en particulier des fibres kératiniques humaines, et plus particuluèrement des cheveux. Ce procédé consiste à appliquer sur les fibres au moins une composition telle que définie ci-dessus contenant, dans un milieu approprié pour la teinture au moins une base d'oxydation selon l'invention, et au moins un polymère de l'invention ; et au moins une composition oxydante contenant au moins un agent oxydant, qui est mélangée juste avant l'emploi à la composition colorante ou qui est appliquée séquentiellement sans rinçage intermédiaire.

Une variante de ce procédé consiste à appliquer une composition formée par une composition A contenant au moins une base d'oxydation selon l'invention, et par une composition B contenant au moins un polymère hydrosoluble sous forme dispersée ou hydrosolubilisée selon l'invention, la composition B étant appliquée avant la composition A ou inversement, la composition selon l'invention étant ainsi formée directement sur les fibres et ensuite suit l'application d'une troisième composition oxydante contenant au moins un agent oxydant.

Le temps d'application est compris généralement entre 3 et 60 minutes et préférentiellement entre 5 à 40 minutes. La température d'application est comprise de préférence entre la température ambiante et 80°C, préférentiellement entre 25°C et 55°C.

L'invention concerne également un dispositif à 2 compartiments pour la teinture des fibres kératiniques en particulier de fibres kératiniques humaines et plus particulièrement les cheveux, tel qu'un premier compartiment renferme une composition colorante contenant, dans un milieu approprié pour la teinture, au moins une base d'oxydation selon l'invention, et que l'autre compartiment renferme une composition contenant au moins un polymère hydrosoluble sous forme de dispersée ou solubilisée et de préférence sous forme dispersée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité. Ainsi, une fois les deux compositions renfermées dans ce dispositif mélangées, on obtient la composition selon l'invention.

L'invention concerne également un dispositif à 2 compartiments pour la teinture des fibres kératiniques en particulier de fibres kératiniques humaines et plus particulièrement les cheveux, tel qu'un premier compartiment renferme une composition colorante contenant, dans un milieu approprié pour la teinture, au moins une base d'oxydation selon l'invention et au moins un polymère hydrosoluble sous forme dispersée ou solubilisée et de préférence sous forme dispersée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité, et que l'autre compartiment renferme une composition oxydante contenant au moins un agent oxydant.

L'invention concerne également un dispositif à 3 compartiments pour la teinture des fibres kératiniques en particulier des fibres kératiniques humaines et plus particulièrement les cheveux, tel qu'un premier compartiment renferme une composition colorante contenant, dans un milieu approprié pour la teinture, au moins une base d'oxydation selon l'invention ; le second compartiment renferme au moins un polymère hydrosoluble sous forme dispersée ou solubilisée et de préférence sous forme dispersée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité, et qu'un troisième compartiment renferme une composition oxydante.

## Revendications

1. Composition pour la teinture des fibres kératiniques, en particulier des fibres kératiniques humaines telles que les cheveux humains contenant au moins une base d'oxydation et au moins un polymère hydrosoluble sous forme dispersée ou sous forme solubilisée de PM moyen en poids supérieur à 10⁶, ledit polymère étant obtenu et introduit dans la composition sous forme d'une dispersion issue de la polymérisation dans une solution aqueuse saline d'au moins un monomère hydrosoluble comportant au moins une double liaison en présence :
- d'au moins un agent dispersant constitué par un (poly)électrolyte soluble, et
- d'au moins un agent empêchant l'augmentation de la viscosité, ladite base d'oxydation étant choisie parmi les paraphénylènediamines substituées, les paraaminophénols substitués, les bases hétérocycliques et leurs sels d'addition avec un acide.

2. Composition selon la revendication 1, **caractérisée par le fait que** la paraphénylènediamine substituée est de formule I : 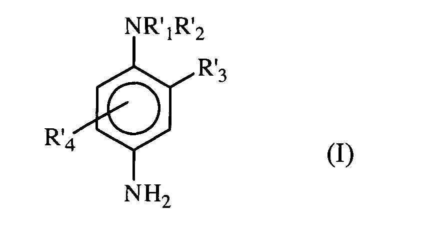 dans laquelle
R'₁, R'₂ représentent indépendamment l'un de l'autre, un atome d'hydrogène, un radical alkyle linéaire ou ramifié en C₁-C₄, un radical mono ou polyhydroxyalkyle linéaire ou ramifié en C₁-C₄, un radical alcoxy(C₁-C₄)alkyle(C₁-C₄) linéaire ou ramifié, ou forme avec l'atome d'azote les portant un hétérocycle éventuellement substitué par un ou plusieurs groupes hydroxyalkyle en C₁-C₄,
R'₃ et R'₄ représentent indépendamment l'un de l'autre, un atome d'hydrogène, un atome d'halogène, un radical alkyle linéaire ou ramifié en C₁-C₄, un radical mono ou polyhydroxyalkyle linéaire ou ramifié en C₁-C₄, un radical alcoxy en C₁-C₄,
l'un au moins des substituants R'₁ à R'₄ étant différent de l'hydrogène.

3. Composition selon la revendication 2, **caractérisée par le fait que** la paraphénylènediamine substituée de formule I est choisie parmi la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

4. Composition selon la revendication 1 ou 2, **caractérisée par le fait que** le paraaminophénol substitué est de formule II : 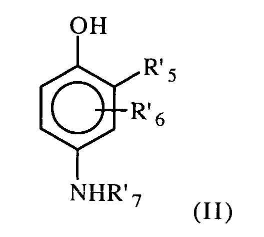 dans laquelle :
R'₅ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
R'₆ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄, ou alcoxy(C₁-C₄)alkyle(C₁-C₄),
étant entendu que l'un au moins un des radicaux R'₅, R'₆ ou R'₇ de préférence R'₅ ou R'₆, est différent d'un atome d'hydrogène.
R'₇ représente un atome d'hydrogène ou un radical alkyle en C₁-C₄,
étant entendu que l'un au moins des radicaux R'₅, R'₆ ou R'₇ de préférence R'₅ ou R'₆, est différent d'un atome d'hydrogène.

5. Composition selon la revendication 4, **caractérisée par le fait que** le paraaminophénol substitué est choisi parmi le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition.

6. Composition selon la revendication 4, **caractérisée par le fait que** le paraaminophénol substitué est le N-méthylparaaminophénol.

7. Composition selon la revendication 1, **caractérisée en ce que** la composition contient au moins une base hétérocyclique choisie parmi les dérivés pyridiniques, les dérivés pyrimidiniques, les dérivés pyrazoliques et leurs sels d'addition avec un acide.

8. Composition selon la revendication 1, **caractérisée par le fait qu'**elle comprend au moins un coupleur.

9. Composition selon la revendication 8, **caractérisée en ce que** les coupleurs sont choisis parmi les méta-phénylènediamines, les méta-aminophénols, les dérivés mono- ou poly-hydroxylés du naphtalène, le sésamol et ses dérivés , les coupleurs indoliques, les coupleurs indoliniques, les coupleurs pyridiniques et leurs sels d'addition avec un acide.

10. Composition selon la revendication 9, **caractérisée en ce que** les sels d'addition avec un acide des bases d'oxydation et coupleurs sont notamment choisis parmi les chlorhydrates, les bromhydrates, les sulfates et les tartrates, les lactates et les acétates.

11. Composition selon l'une quelconque des revendications 1 à 10, **caractérisée en ce que** le ou les monomères hydrosolubles comportant au moins une double liaison sont choisis parmi des monomères cationiques, anioniques, non anioniques et leurs mélanges.

12. Composition selon la revendication 11, **caractérisée en ce que** les monomères anioniques sont choisis parmi l'acide (méth)acrylique, l'acide acrylamido-2-méthylpropanesulfonique, l'acide itaconique et leurs sels d'un métal alcalin, d'alcalinoterreux ou d'ammonium ou ceux issus d'une amine organique telle que l'alcanolamine.

13. Composition selon la revendication 11, **caractérisée en ce que** les monomères non ioniques sont choisis parmi le (méth)acrylamide, le N-vinylformamide, le N-vinylacétoamide et le (méth)acrylate d'hydroxypropyle.

14. Composition selon la revendication 11, **caractérisée en ce que** les monomères cationiques sont choisis parmi les sels d'ammonium quaternaire dérivés d'une diallylamine, et ceux répondant à la formule suivante (III) : 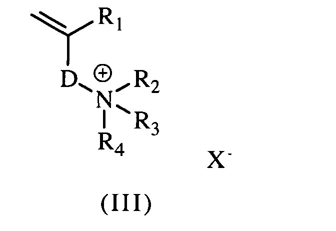 dans laquelle :
R₁ représente un atome d'hydrogène ou un groupement méthyle,
R₂ et R₃, identiques ou différents, représentent un atome d'hydrogène ou un groupe alkyle en C₁-C₄ linéaire ou ramifié,
R₄ représente un atome d'hydrogène, un groupe alkyle en C₁-C₄ linéaire ou ramifié, un groupe aryle,
D représente le motif suivant, 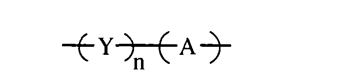
dans lequel :
Y représente une fonction amide, un ester : O-CO ou CO-O, un uréthane ou une urée,
A représente un groupement alkylène en C₁-C₁₀ linéaire ou ramifié, cyclique ou acyclique, pouvant être substitué par un groupement aromatique ou hétéroaromatique, les groupements alkylènes pouvant être interrompus par un groupement aromatique ou hétéroaromatique divalent, un atome d'oxygène, un atome d'azote, un atome de soufre ou un atome de phosphore ; l'alkylène pouvant contenir une cétone, un amide, un ester : O-CO ou CO-O, un uréthane, ou une urée,
n est un nombre entier variant de 0 à 1,
X⁻ représente un contre ion anionique, comme un chlorure ou un sulfate.

15. Composition selon l'une quelconque des revendications précédentes, **caractérisée en ce que** le polymère hydrosoluble est polymérisé à partir d'au moins un monomère cationique.

16. Composition selon l'une quelconque des revendications précédentes, **caractérisée en ce que** le polymère hydrosoluble est polymérisé à partir des monomères comportant au moins une double liaison suivants :
- 0 à 30% en moles d'acide acrylique,
- 0 à 95,5% en moles d'acrylamide et,
- 0,5 à 100% en moles d'au moins un monomère cationique de formule (III) telle que définie dans la revendication 13.

17. Composition selon la revendication 16, **caractérisée en ce que** le polymère hydrosoluble est polymérisé à partir d'un monomère cationique et d'acide acrylique, le nombre de mole du monomère cationique étant supérieur au nombre de mole d'acide acrylique.

18. Composition selon la revendication 16, **caractérisée en ce que** le polymère hydrosoluble est polymérisé à partir de 10% de chlorure d'acryloyloxyéthyldiméthylbenzylammonium et de 90% d'acrylamide.

19. Composition selon la revendication 16, **caractérisée en ce que** le polymère hydrosoluble est polymérisé à partir de 30% de chlorure d'acryloyloxytriméthylammonium, 50% de chlorure d'acryloyloxyéthyldiméthylbenzylammonium, et de 20 % d'acrylamide,

20. Composition selon la revendication 16, **caractérisée en ce que** le polymère hydrosoluble est polymérisé à partir de 10% de chlorure d'acryloyloxyéthyltriméthylammonium et de 90% d'acrylamide,

21. Composition selon la revendication 16, **caractérisée en ce que** le polymère hydrosoluble est polymérisé à partir de 30% de chlorure de diallyldiméthylammonium et de 70% d'acrylamide,

22. Composition selon l'une quelconque des revendications 1 à 13, **caractérisée en ce que** le polymère hydrosoluble est polymérisé à partir de 30% d'acide acrylique et de 70% d'acrylamide.

23. Composition selon l'une quelconque des revendications précédentes, **caractérisée en ce que** l'agent dispersant est un polyélectrolyte cationique obtenu par polymérisation de 50 à 100% en moles d'au moins un monomère cationique choisi parmi les sels dérivés du (méth)acrylate de diméthylaminoéthyle, du N-[diméthylaminopropyl]-(méth)acrylamide, ou d'une di(méth)allylamine, le chlorure de (méth)acryloyloxytriméthylammonium, le chlorure de (méth)acrylamido-propyltriméthylammonium, le chlorure de diméthyldiallylammonium, et leurs mélanges, et de 50 à 0% en moles d'acrylamide.

24. Composition selon l'une quelconque des revendications précédentes, **caractérisée en ce que** la solution aqueuse saline comprend au moins un sel anionique divalent.

25. Composition selon la revendication 24, **caractérisée en ce que** le sel anionique divalent est choisi parmi le sulfate d'ammonium, l'hydrogénosulfate d'ammonium, le sulfate de sodium, l'hydrogénosulfate de sodium, le sulfate de magnésium, l'hydrogénosulfate de magnésium, le sulfate d'aluminium, l'hydrogénosulfate d'aluminium.

26. Composition selon l'une quelconque des revendications précédentes, **caractérisée en ce que** l'agent empêchant l'augmentation de la viscosité du milieu réactionnel lors de la polymérisation est choisi parmi :
(A). les acides polycarboxyliques ou leurs sels,
(B). les polyphénols,
(C). les composés cycliques contenant un groupe hydroxy et un groupe carboxy, ou leurs sels,
(D). l'acide gluconique ou ses sels,
(E). les produits réactionnels obtenus par réaction d'une méthoxyhydroquinone et/ou d'un monomère cationique (méth)acrylique avec un composé qui génère des radicaux, sous une atmosphère oxydante,
(F). les produits réactionnels obtenus par réaction d'un polymère cationique (méth)acrylique avec un composé qui génère des radicaux, sous une atmosphère oxydante,
(G). les produits réactionnels obtenus par réaction d'un polymère cationique (méth)acrylique avec un oxydant, et
leurs mélanges.

27. Composition selon la revendication 26, **caractérisée en ce que** l'agent empêchant l'augmentation de la viscosité (A) est choisi parmi l'acide oxalique, l'acide adipique, l'acide tartrique, l'acide malique, l'acide phtalique et leurs sels.

28. Composition selon la revendication 26, **caractérisée en ce que** l'agent empêchant l'augmentation de la viscosité (B) est choisi parmi le résorcinol et le pyrogallol.

29. Composition selon la revendication 26, **caractérisée en ce que** l'agent empêchant l'augmentation de la viscosité (C) est choisi parmi l'acide m-hydroxybenzoïque, l'acide p-hydroxybenzoïque, l'acide salicylique, l'acide gallique, l'acide tannique et leurs sels.

30. Composition selon la revendication 26, **caractérisée en ce que** l'agent empêchant l'augmentation de la viscosité (D) est choisi parmi le gluconate de sodium, le gluconate de potassium, le gluconate d'ammonium, et différents sels aminés de l'acide gluconique.

31. Composition selon l'une quelconque des revendications précédentes, **caractérisée en ce que** la ou les bases d'oxydation représentent de préférence, de 0,0005% à 12% en poids environ du poids total de la composition et encore plus préférentiellement de 0,005% à 6% en poids environ.

32. Composition selon l'une quelconque des revendications de 7 à 30, **caractérisée en ce que**, le ou les coupleurs représentent de préférence, de 0,0001% à 10% en poids environ du poids total de la composition et encore plus préférentiellement de 0,005% à 5% en poids environ.

33. Composition selon l'une quelconque des revendications précédentes, **caractérisée en ce que** le ou les polymères de l'invention sont présents à une concentration comprise entre 0,05% et 10%, de préférence entre 0,1% et 5%, et encore plus préférentiellement entre 0,2% et 2% en poids par rapport au poids total de la composition.

34. Composition selon l'une quelconque des revendications précédentes, **caractérisée en ce que** la composition contient un ou des colorants directs.

35. Composition selon la revendication 34, **caractérisée en ce que** le ou les colorants directs utilisés selon l'invention représentent de préférence, de 0,001% à 10% en poids environ du poids total de la composition tinctotriale et encore plus préférentiellement de 0,005% à 5% en poids environ.

36. Composition selon l'une quelconque des revendications précédentes, **caractérisée en ce que** le milieu approprié pour la composition pour la coloration de fibres kératiniques est de préférence un milieu aqueux constitué par de l'eau ou par un mélange d'eau et d'un solvant cosmétiquement acceptable.

37. Composition selon la revendication 36, **caractérisée en ce que** le solvant approprié est choisi parmi les alcools inférieurs en C₁-C₄, l'alcool benzylique, les éthers de polyols, les alcanes en C₅-C₁₀, les cétones en C₃-C₄, les acétates d'alkyles en C₁-C₄, le diméthoxyéthane, et leurs mélanges.

38. Composition selon l'une quelconque des revendications précédentes, telle qu'elle comprend des additifs cosmétiques choisis parmi des agents tensio-actifs anioniques, cationiques, non-ioniques ou amphotères, des agents épaississants non polymériques comme des acides ou des électrolytes, des agents antioxydants, des agents dispersants, des agents séquestrants, des parfums, des agents de conditionnement, des agents conservateurs, des agents opacifiants, des agents alcalins, des agents acides.

39. Composition selon l'une quelconque des revendications précédentes, **caractérisée en ce que** le polymère de PM moyen supérieur à 10⁶ est présent sous forme solubilisée.

40. Composition selon l'une quelconque des revendications précédentes, **caractérisée en ce qu'**elle se présente sous la forme d'une lotion, d'une crème, d'un gel.

41. Composition selon l'une quelconque des revendications précédentes, **caractérisée en ce qu'**elle est conditionnée en aérosol.

42. Composition selon l'une quelconque des revendications précédentes, **caractérisée en ce qu'**elle contient un oxydant, et plus particulièrement du peroxyde d'hydrogène.

43. Procédé de préparation de composition **caractérisé par le fait qu'**il consiste à introduire dans un milieu contenant au moins une base d'oxydation choisie parmi les paraphénylènediamines substituées, les paraaminophénols substitués et les bases hétérocycliques, et au moins une dispersion d'un polymère hydrosoluble de PM moyen en poids supérieur à 10⁶, ladite dispersion étant obtenue par polymérisation dans une solution aqueuse saline d'au moins un monomère hydrosoluble comportant au moins une double liaison en présence :
- d'au moins un agent dispersant constitué par un (poly)électrolyte soluble,
- et d'au moins un agent empêchant l'augmentation de la viscosité.

44. Procédé de préparation de composition **caractérisé par le fait qu'**il consiste à introduire dans un milieu cosmétiquement acceptable au moins une dispersion contenant au moins une base d'oxydation telle que définie dans les revendications 1 à 6 et au moins un polymère hydrosoluble de PM moyen en poids supérieur à 10⁶, ladite dispersion étant obtenue par polymérisation dans une solution aqueuse saline d'au moins un monomère hydrosoluble comportant au moins une double liaison en présence :
- d'au moins un agent dispersant constitué par un (poly)électrolyte soluble,
- et d'au moins un agent empêchant l'augmentation de la viscosité.

45. Procédé de teinture des fibres kératiniques et en particulier fibres kératiniques humaines, et plus particulièrement les cheveux, **caractérisé par le fait qu'**il consiste à appliquer sur les fibres au moins une composition telle que définie dans les revendications 1 à 41, et une composition oxydante contenant au moins un agent oxydant, qui est mélangée juste au moment de l'emploi à la composition colorante ou qui est appliquée séquentiellement sans rinçage intermédiaire.

46. Procédé selon la revendication 44 **caractérisé en ce que** le temps d'application est compris généralement entre 3 et 60 minutes et préférentiellement entre 5 à 40 minutes ; la température d'application étant comprise de préférence entre la température ambiante et 80°C préférentiellement entre 25°C et 55°C.

47. Procédé de teinture des fibres kératiniques et en particulier fibres kératiniques humaines, et plus particulièrement les cheveux, **caractérisé par le fait qu'**il consiste à appliquer sur les fibres une composition formée par une composition A contenant au moins une base d'oxydation, et par une composition B contenant au moins un polymère hydrosoluble sous forme dispersée ou solubilisée telle que définie dans les revendications 1 et 11 à 30 ; la composition A étant appliquée sur les fibres avant la composition B, et à appliquer ensuite une composition oxydante contenant au moins un agent oxydant.

48. Procédé de teinture des fibres kératiniques et en particulier fibres kératiniques humaines, et plus particulièrement les cheveux, **caractérisé par le fait qu'**il consiste à appliquer sur les fibres une composition formée par une composition A contenant au moins une base d'oxydation selon l'invention, et par une composition B contenant au moins un polymère hydrosoluble sous forme de solubilisée ou dispersée telle que définie dans les revendications 1 et 11 à 30 ; la composition B étant appliquée sur les fibres avant la composition A, et à appliquer ensuite une composition oxydante contenant au moins un agent oxydant.

49. Dispositif à 2 compartiments pour la teinture des fibres kératiniques en particulier de fibres kératiniques humaines et plus particulièrement les cheveux, **caractérisé par le fait qu'**un premier compartiment renferme une composition colorante contenant, dans un milieu approprié pour la teinture, au moins une base d'oxydation, et que l'autre compartiment renferme une composition contenant au moins un agent oxydant et au moins un polymère sous forme dispersée ou solubilisée, et de préférence sous forme dispersée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité.

50. Dispositif à 2 compartiments pour la teinture des fibres kératiniques en particulier de fibres kératiniques humaines et plus particulièrement les cheveux, **caractérisé par le fait qu'**un premier compartiment renferme une composition colorante contenant, dans un milieu approprié pour la teinture, au moins une base d'oxydation et au moins un polymère hydrosoluble sous forme dispersée ou solubilisée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité, et que l'autre compartiment renferme une composition oxydante, contenant au moins un agent oxydant.

51. Dispositif à 3 compartiments pour la teinture des fibres kératiniques en particulier de fibres kératiniques humaines et plus particulièrement les cheveux, **caractérisé par le fait qu'**un premier compartiment renferme une composition colorante contenant, dans un milieu approprié pour la teinture, au moins une base d'oxydation, un second compartiment renferme au moins un polymère hydrosoluble sous forme dispersée ou solubilisée et de préférence sous forme dispersée de PM moyen en poids supérieur à 10⁶, ledit polymère étant obtenu par polymérisation dans une solution aqueuse saline d'au moins un monomère hydrosoluble comportant au moins une double liaison contenant au moins un agent dispersant constitué par un (poly)électrolyte soluble, et au moins un agent empêchant l'augmentation de la viscosité, et qu'un troisième compartiment renferme une composition oxydante, contenant au moins un agent oxydant.
